# EUROPÄISCHE PATENTSCHRIFT

(11) **EP 1 051 421 B1**
(45) Veröffentlichungstag und Bekanntmachung des Hinweises auf die Patenterteilung: **11.12.2002**
(21) Anmeldenummer: 99914394.4
(22) Anmeldetag: 27.01.1999
(51) Int. Cl.: C07F 5/02, A61K 41/00

(54) **BOR-HALTIGE VERBINDUNGEN FÜR ELEKTRONENMIKROSKOPIE UND FÜR BOR-NEUTRONENEINFANGTHERAPIE**
COMPOUNDS CONTAINING BORON FOR ELECTRON MICROSCOPY AND FOR BORON NEUTRON-CAPTURE THERAPY
COMPOSES CONTENANT DU BORE POUR LA MICROSCOPIE ELECTRONIQUE ET POUR LA NEUTRONTHERAPIE INITIALE AU BORE

(30) Priorität: 28.01.1998 DE 19803206
(43) Veröffentlichungstag der Anmeldung: 15.11.2000
(73) Patentinhaber: Deutsches Krebsforschungszentrum Stiftung des öffentlichen Rechts, 69120 Heidelberg (DE)
(72) Erfinder: WIESSLER, Manfred, D-67227 Frankenthal (DE); TRÖSTER, Helmut, D-68165 Mannheim (DE); RADDATZ, Stefan, D-69115 Heidelberg (DE); SPIESS, Eberhard, D-68526 Ladenburg (DE); TRENDELENBURG, Michael, D-69221 Dossenheim (DE)
(74) Vertreter: Schüssler, Andrea, Dr.
(86) Internationale Anmeldenummer: DE9900257
(87) Internationale Veröffentlichungsnummer: WO99038870

(56) Entgegenhaltungen:
- CHEMICAL ABSTRACTS, vol. 125, no. 15, 7. Oktober 1996 Columbus, Ohio, US; abstract no. 195765, JIANG, WEI ET AL: "Carboracycles: Macrocyclic Compounds Composed of Carborane Icosahedra Linked by Organic Bridging Groups" XP002106532 & INORG. CHEM. (1996), 35(19), 5417-5426 CODEN: INOCAJ;ISSN: 0020-1669,1996,
- QUALMANN, B. ET AL.: "synthesis of boron-rich lysine dendrimers as protein labels in electron microscopy" ANGEW. CHEM. INT. ED. ENGL., Bd. 35, Nr. 8, 1996, Seiten 909-911, XP002106531 in der Anmeldung erwähnt

## Beschreibung

Die Erfindung betrifft Bor-haltige Verbindungen, deren Herstellung und deren Verwendung für die energiefilternde Transmissionselektronenmikroskopie und für die Bor-Neutroneneinfangtherapie.

Es ist bekannt, Bor-haltige Verbindungen mit der energiefilternden Transmissionselektronenmikroskopie (EFTEM) nachzuweisen.

Beispielsweise beschreibt Qualmann B. et al., *Angew. Chem*., **1996**, *180*, Seiten 970 bis 973 die Synthese Bor-haltiger Lysindendrimere zur Proteinmarkierung in der Elektronenmikroskopie. Die in dieser Veröffentlichung beschriebene Bor-haltige Verbindung zur Markierung von Proteinen ist wegen ihrer großen Ausdehnung zur Markierung von kleinen biologischen Molekülen, z.B. Oligonukleotiden, ungeeignet, da die Stoffeigenschaften solcher kleiner biologischer Moleküle zu stark verändert werden. Außerdem ist die Bor-Dichte durch die große Ausdehnung der Verbindung und durch die Anordnung der 1,2-Dicarbadodecarboran-Fragmente (Carborane) in der äußersten Sphäre des Moleküls sehr gering. Der Einzelnachweis dieser Verbindung mit EFTEM ist deswegen nicht befriedigend möglich. Desweiteren hat die beschriebene Verbindung ein peptidisches Grundgerüst mit L-Lysin als Bausteine, so daß sie für einen enzymatischen Abbau anfällig ist.

Die Publikation von Newkome G.R. et al., in *Angew. Chem.* **1994**, *106*, Seiten 701 bis 703 beschreibt unimolekulare Micellen, die 4 bzw.12 Carborane im Inneren der Micellen sowie hydrophile Gruppen an der Oberfläche der Micellen enthalten. Diese unimolekularen Micellen sind ebenfalls zu groß. Außerdem existiert keine Bindungsstelle für die Ankopplung eines Spacers zur Anbindung an Biomoleküle, wie Oligonukleotide und Proteine.

Daher war es bisher nicht möglich, kleine biologisch wirksame Substanzen, wie Oligonukleotide, für den Nachweis durch die EFTEM durch einen kovalent zu bindenden Bor-haltige Verbindung zu markieren.

Ferner ist es bekannt, borhaltige Verbindungen in der Bor-Neutroneneinfangtherapie einzusetzen, wobei es noch nicht gelang, ausreichend hohe Bor-Konzentrationen selektiv in das Tumorgewebe einzubringen.

Somit liegt der Erfindung die Aufgabe zugrunde, eine Verbindung bereitzustellen, die nicht die Nachteile des Standes der Technik aufweist.

Erfindungsgemäß wird dies durch die Gegenstände in den Patentansprüchen erreicht.

Gegenstand der vorliegenden Erfindung ist eine Bor-haltige Verbindung, die die folgende allgemeine Formel (1) aufweist, 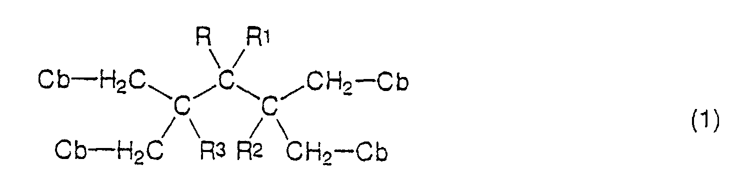 in der
Cb für ein Carboran,
R² und R³ unabhängig voneinander für ein Wasserstoffatom oder einen organischen Rest und
R und R¹ unabhängig voneinander für ein Wasserstoffatom oder eine organische Gruppe stehen oder R und R¹ mit dem Kohlenstoffatom, an das sie gebunden sind, eine Carbonylgruppe bilden.

Der Ausdruck "Carboran" umfaßt Verbindungen jeglicher Art, die die Summenformel B₁₀C₂H₁₂ aufweisen. Die Carborane können drei Isomere bilden: 1,2-Dicarba-*closo*-dodecaboran(12), 1,7-Dicarba-*closo*-dodecaboran(12) und 1,12-Dicarba-*closo*-dodecaboran(12), die auch als ortho-, metha- und para-Carboran bezeichnet werden. Von diesen Isomeren ist das 1,2-Dicarba-*closo*-dodecaboran(12) bevorzugt, das wie folgt symbolisiert wird:

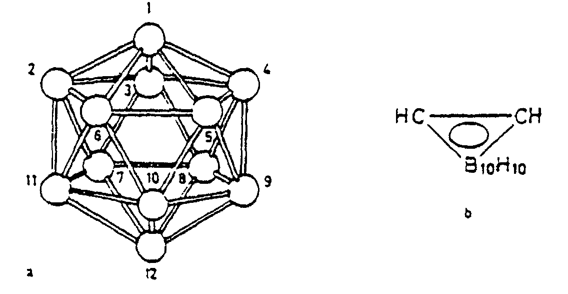

Durch die beiden Kohlenstoffatome weisen die Carborane zwei mögliche Bindungsstellen mit anderen Verbindungen auf. Durch die Verknüpfung der vier Carborane mit dem in der Formel (1) angegebenen Kohlenwasserstoffgerüst ist eine Bindungsstelle des Carborans besetzt. Die zweite Bindungsstelle kann nun zum Anbinden von Spacern und/oder löslichkeitsmodulierenden Verbindungen verwendet werden.

Der Ausdruck "Spacer" umfaßt Verbindungen jeglicher Art, die zur Verknüpfung, insbesondere zur kovalenten Verknüpfung, der erfindungsgemäßen Bor-haltigen Verbindung mit anderen Molekülen, z.B. mit biologischen Molekülen, eingesetzt werden können. Solche Verbindungen sind dem Fachmann bekannt. Vorzugsweise handelt es sich um von C2 bis C10 Alkane, insbesondere C6 Alkane abgeleitete Verbindungen, die vorzugsweise linear sind und die ggf. Etherbrücken aufweisen oder über eine solche an das Carboran gebunden sein können. Der Ausdruck "biologische Moleküle" weist darauf hin, daß es sich um für biologische Prozesse relevante Moleküle jeglicher Art handelt. Beispiele sind Proteine, Nukleotide, wie Mono-, Oligo-, und Polynukleotide, Nukleoside, Nukleosiddiphosphate und Nukleosidtriphosphate. Von den Proteinen sind solche bevorzugt, die sich in Tumoren anreichern, wie Albumin.

Bei den "löslichkeitsmodulierenden Verbindungen" handelt es sich um Verbindungen jeglicher Art, die die Löslichkeit der erfindungsgemäßen Verbindung in einem Lösungsmittel, insbesondere Wasser oder einem wäßrigen Lösungsmittel, erhöhen oder erniedrigen. Zur Erhöhung der Wasserlöslichkeit können die löslichkeitsmodulierenden Verbindungen mindestens eine polare Gruppe, wie eine Hydroxylgruppe, aufweisen. Beispiele dafür sind -CH₂OH und Polyhydroxyverbindungen, wie Inositol oder Saccharide, insbesondere Monosaccharide, vorzugsweise Glucose. Wegen der hohen Lipophilie der Carborane und des Kohlenwasserstoffgerüsts ist die Anbindung löslichkeitsmodulierender Verbindungen für die Erhöhung der Wasserlöslichkeit der Bor-haltigen Verbindung günstig.

Durch die Verwendung von Kohlenhydraten, insbesondere Glucose, Galactose, Xylose, Fucose oder auch Gentobiose, kann weiterhin die Tumorselektivität der erfindungsgemäßen Bor-haltigen Verbindung erhöht werden.

Beispiele von Verbindungen, bei denen an die Carborane Glucose gebunden ist, sind die in den Formeln (2) und (3) dargestellten Verbindungen.

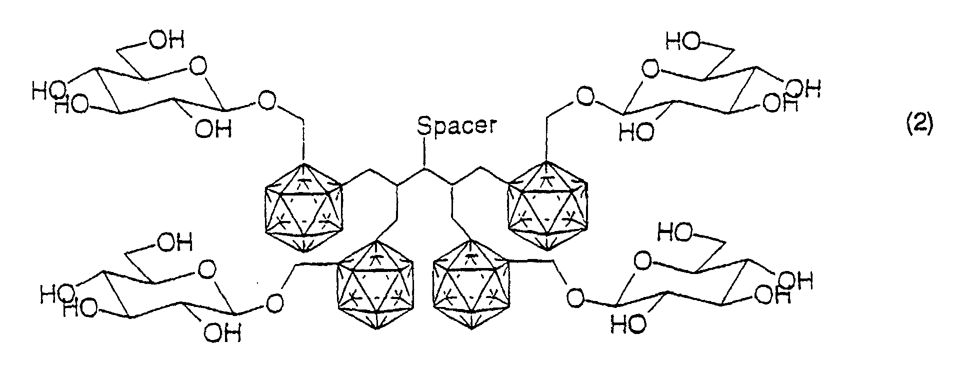

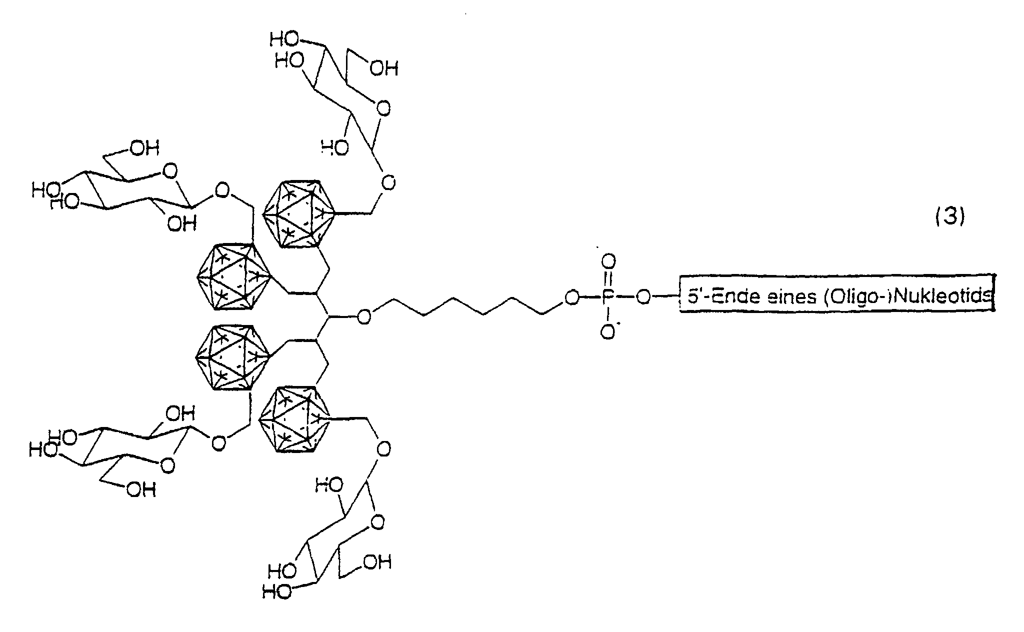

Auf diese Weise werden kleine lipophile Molekülkerne erhalten, in denen die gesamte Bormenge konzentriert ist, sowie eine hydrophile Molekülhülle, die die Wasserlöslichkeit der Bor-haltigen Verbindung und ggf. die Tumorselektivität erhöht. Insgesamt wird somit eine unimolekulare Micelle erhalten.

In der erfindungsgemäßen Bor-haltigen Verbindung stehen die Substituenten R² und R³ unabhängig voneinander für ein Wasserstoffatom oder einen organischen Rest. Der Ausdruck "organischer Rest" umfaßt organische Verbindungen jeglicher Art, die Kohlenstoff, Wasserstoff und ggf. Sauerstoff, Schwefel, Phosphor und Bor umfassen. Beispiele hierfür sind die Gruppen -NO₂, -(C=O)-, -C≡N-, Phenyl- und -COOR⁴, wobei R⁴ z.B. für einen Alkylrest, wie Ethyl, steht. R² und R³ können auch miteinander verbunden sein, d.h. die für R² und R³ stehenden Reste werden so gewählt, daß sie mit den Kohlenstoffatomen, an die sie direkt gebunden sind, sowie dem die Reste R und R¹ aufweisenden Kohlenstoffatom einen Ring, vorzugsweise einen 6-gliedrigen Ring, bilden. An den Ring können weitere Carborane gebunden werden, so daß die Gesamtzahl der Carborane in der erfindungsgemäßen Verbindung erhöht wird, z.B. auf 6. Beispiele für R² und R³ zur Ausbildung eines 6-gliedrigen Rings sind:

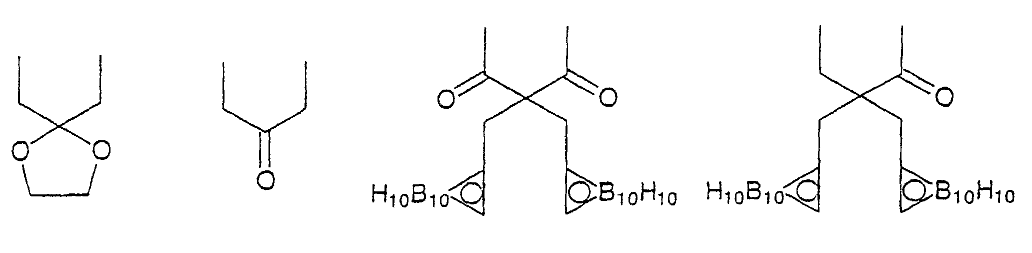

Die Substituenten R und R¹ stehen in der erfindungsgemäßen Bor-haltigen Verbindung unabhängig voneinander für ein Wasserstoffatom, eine organische Gruppe oder R und R¹ bilden zusammen mit dem Kohlenstoffatom, an das sie gebunden sind, eine Carbonylgruppe. Die organische Gruppe kann jede Kohlenstoff, Wasserstoff und ggf. Sauerstoff, Schwefel, Phosphor und Bor enthaltende Verbindung sein. Beispielsweise ist die Gruppe eine über ein Ether-Brücke gebundene C2 bis C10, insbesondere C6, Alkyl-Gruppe. An die Alkylgruppe, insbesondere an deren Ende, kann eine Phosphatgruppe (PO₄³⁻) gebunden sein. Die Phosphatgruppe kann mit einem biologischen Molekül, wie einem Poly-, Oligo- oder Mononukleotid, verbunden sein, vorzugsweise an deren 5'-Enden. Ein Beispiel einer solchen für R bzw. R¹ stehenden organischen Gruppe ist in der vorstehenden Formel (3) dargestellt.

Ein weiterer Gegenstand der vorliegenden Erfindung ist ein Verfahren zur Herstellung der erfindungsgemäßen Bor-haltigen Verbindungen, bei dem ein Decaboran(14) mit einem Alkin der Formel (4) umgesetzt wird.

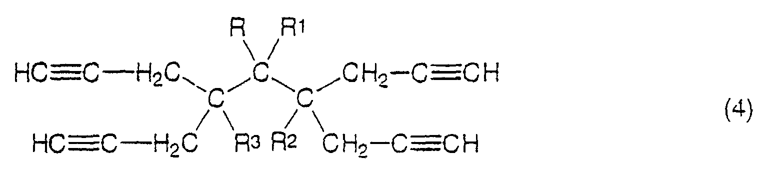

Bei einem Decaboran handelt es sich um eine Verbindung mit der Summenformel B₁₀H₁₄, die sich von den vorstehend beschriebenen Carboranen dadurch unterscheidet, daß u.a. die zwei Kohlenstoffatome nicht vorliegen. Diese werden durch die zwei Kohlenstoffatome der Alkin-Bindung eingebracht.

Überraschenderweise wurde gefunden, daß diese Umsetzung auch dann möglich ist, wenn mindestens vier Carborane trotz der sterischen Hinderung an ein sehr kleines Kohlenwasserstoffgrundgerüst gebunden werden. Desweiteren wurde festgestellt, daß überraschend hohe Ausbeuten erhalten werden, wenn sich in β-Stellung zur Alkin-Gruppe eine Carbonylgruppe befindet. Die Carbonylgruppe kann, falls es erforderlich ist, in üblicher Weise geschützt und nachfolgend, ebenfalls in bekannter Weise, entschützt werden.

Beispiele dieser Umsetzung sind in den Reaktionsgleichungen (I) bis (VI) dargestellt:

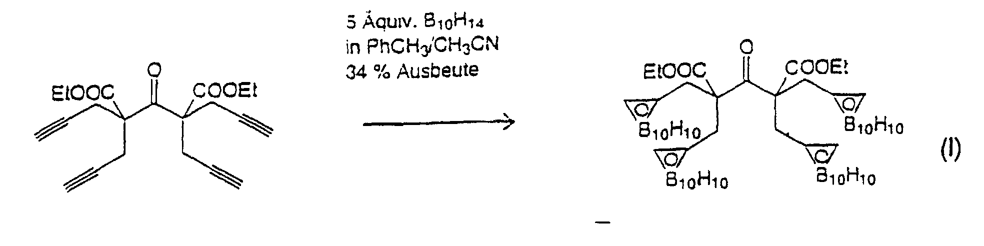

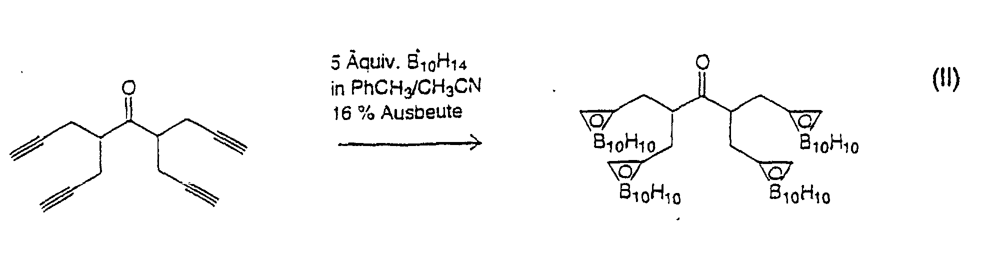

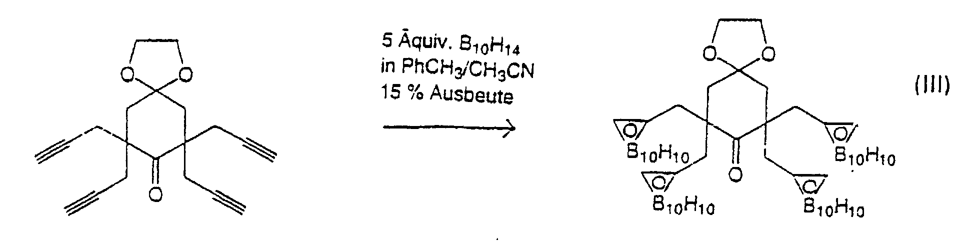

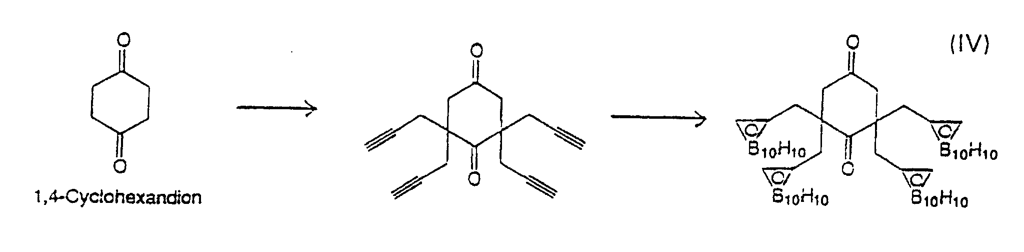

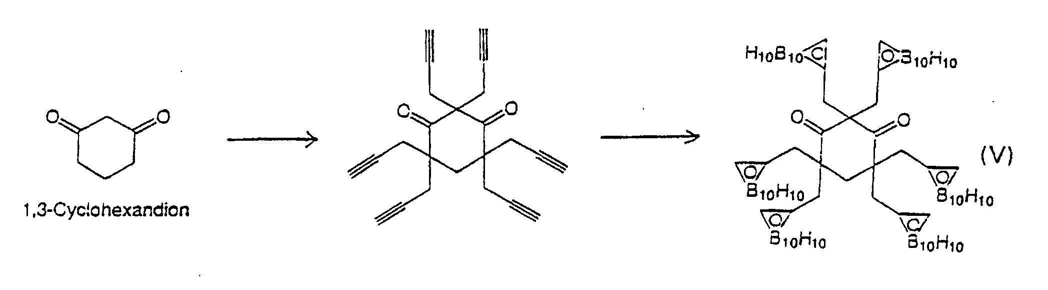

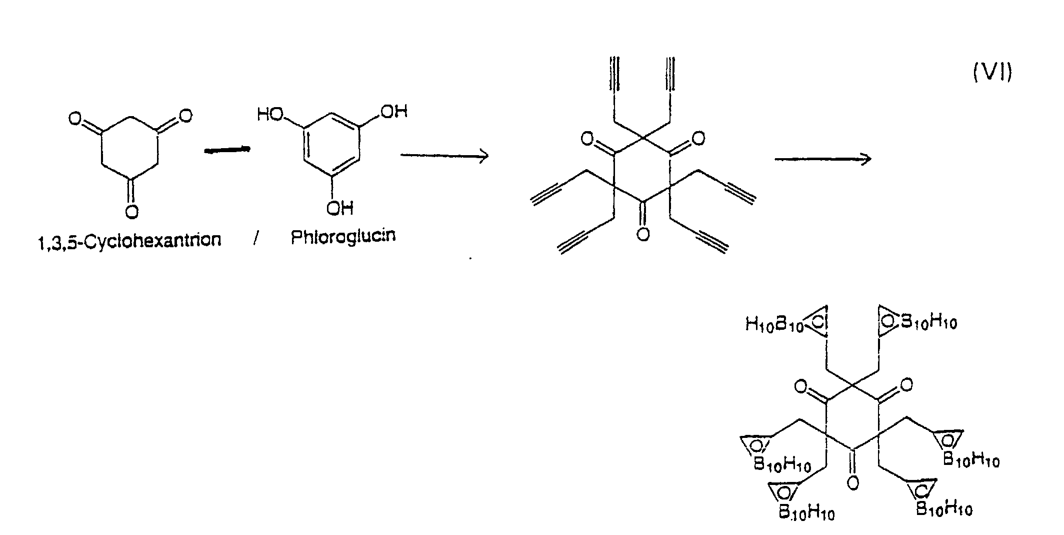

Die Alkinverbindungen können in dem Fachmann bekannter Weise aus CH-aciden Carbonylverbindungen hergestellt werden. Dies geschieht beispielsweise mittels C-Alkylierung von Enolaten von Estern oder Ketonen, die durch Deprotonierung durch geeignete Basen, wie z.B. wäßrige Natronlauge unter Phasentransferkatalyse oder Natriummethylat in Methanol erzeugt werden können und mit einem Proparylhalogenid, wie z.B. Proparylbromid, abreagieren.

Die erhaltenen erfindungsgemäßen Bor-haltigen Verbindungen bieten pro Carboran eine weitere Position zur Einführung der bereits vorstehend erwähnten Spacer oder löslichkeitsmodulierenden Verbindungen, wie Saccharide, insbesondere Glucose. Die Carbonylgruppe(n) kann (können) nachfolgend zur Anbindung eines vorstehend beschriebenen Spacers und damit zur Kopplung an biologische Moleküle und löslichkeitsmodulierende Verbindungen verwendet werden. Hierzu kann die Carbonylgruppe zum Alkohol reduziert werden, z.B. mit LiAlH₄, gefolgt von der Ausbildung einer Ethergruppe.

Für das erfindungsgemäße Verfahren zur Herstellung Bor-haltiger Verbindungen können als Ausgangsverbindungen auch Alkine eingesetzt werden, die bereits Seitenketten für die Ankopplung von biologischen Molekülen, z.B. Sacchariden, aufweisen. Eine solche Ausgangsverbindun ist in der Formel (5) angegeben.

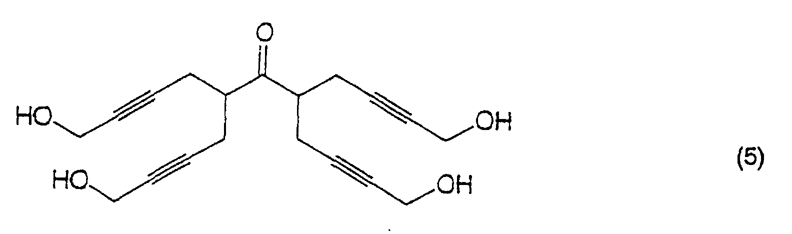

Die Anbindung der biologischen Moleküle kann dabei an die Hydroxylgruppe(n) erfolgen. Es hat sich für diese Reaktion als günstig erwiesen, die vier OH-Gruppen vor der Umsetzung mit dem Decaboran(14) zu schützen und nach der erfolgten Umsetzung zu entschützen. Als Schutzgruppe kann jede für OH-Gruppen bekannte Schutzgruppe, z.B. eine Estergruppe, wie eine Acetatgruppe, eingesetzt werden. Das Entschützen erfolgt in bekannter Weise, abhängig von der verwendeten Schutzgruppe..

Die erfindungsgemäßen Verbindungen weisen eine Reihe von Vorteilen auf: Die räumliche Ausdehnung der erfindungsgemäßen Verbindungen ist sehr gering, so daß Störungen der Eigenschaften der biologischen Moleküle, z.B. der Oligonukleotide, nicht auftreten. Ferner wird durch die Verknüpfung der Carborane durch das enge (kleine) Kohlenwasserstoffgerüst eine hohe Stabilität der Bor-haltigen Verbindung erreicht. Weiterhin ist die erfindungsgemäße Verbindung exakt definierbar und an einem biologischen Molekül eindeutig positionierbar. Außerdem sind die Eigenschaften der Bor-haltigen Verbindungen durch den Spacer und die löslichkeitsmodifizierende Verbindung in großer Breite modifizierbar. Daraus ergibt sich eine vielfältige Verwendbarkeit für die kovalente Bindung an biologische Moleküle, wie Proteine, monomere Nukleosidtriphosphate sowie Oligo- und Polynukleotide, so daß die erfindugsgemäßen Bor-haltigen Verbindungen bestens für die energiefilternde Transmissionselektronenmikroskopie geeignet sind. Die erfindungsgemäßen Bor-haltigen Verbindungen können auch bestens für die Bor-Neutroneneinfangtherapie zur Behandlung von Tumoren verwendet werden, da zum einen eine ausreichende Bor-Dichte erreicht wird und zum anderen durch eine Verknüpfung mit Sacchariden eine hervorragende Tumorselektivität entweder durch Aufnahme über Glucose-Transporter (GLUT's oder SGLT's) oder durch Lektin-vermittelte Endocytose erzeugt werden kann.

### Kurze Beschreibung der Zeichnung:

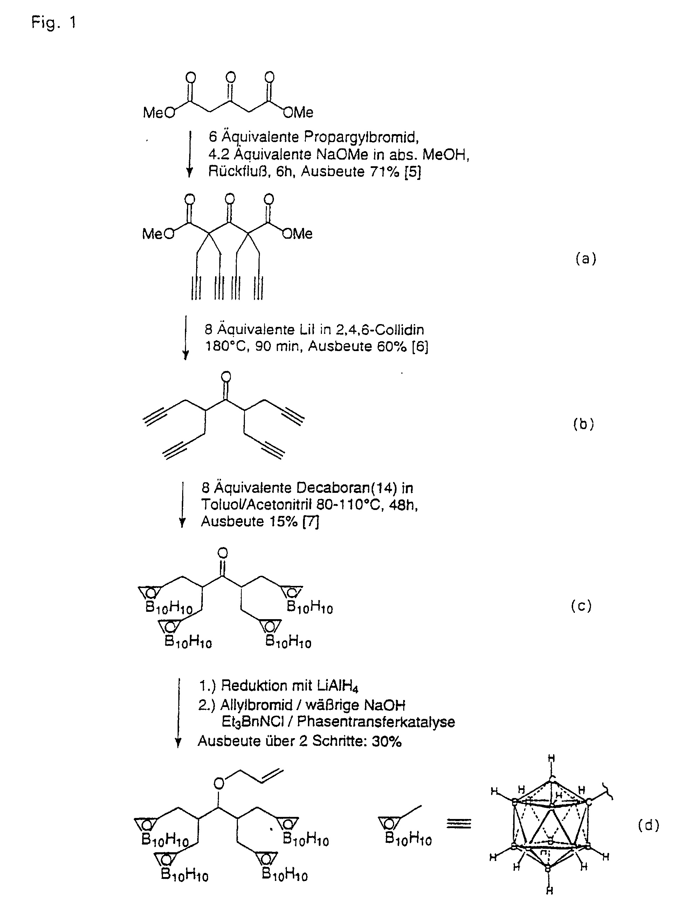

Fig.1 zeigt die Herstellung einer erfindungsgemäßen Bor-haltigen Verbindung.

### Das folgende Beispiel erläutert die Erfindung:

### Beispiel: Herstellung von 2-Allyloxy-1,1,3,3-tetrakis(closo-1,2-di carbadodecaboranylmethyl)-propan

Das Schema zur Herstellung oben genannter Verbindung ist in Fig. 1 gezeigt

### (a) 2,2,4,4-Tetrapropargylacetondicarbonsäuredimethylester

Unter einer Argon-Atmosphäre wurden in trockenem Methanol (Molsieb 3 Å) 238 mg (1,37 mmol) Acetondicarbonsäuredimethylester, 0,90 ml (8,0 mmol) einer 80%igen Lösung von Propargylbromid in Toluol sowie 0,5 ml einer 5,4 molaren (2,7 mmol) Natriummethylat-Lösung in Methanol gelöst. Es wurde 1 h unter Rückfluß erhitzt, anschließend gab man weitere 0,5 ml Natriummethylat-Lösung in Methanol zum Reaktionsgemisch und erhitzte weitere 5 h unter Rückfluß. Man ließ abkühlen und versetzte das Reaktionsgemisch mit verdünnter Salzsäure im Überschuß. Es wurde dreimal mit Diethylether extrahiert, die organischen Phasen wurden vereinigt, mit Wasser gewaschen und über wasserfreiem Natriumsulfat getrocknet. Man entfernte das Lösungsmittel am Rotationsverdampfer und destillierte den Rückstand bei Wasserstrahlvakuum im Kugelrohrofen. Bei 220°C schied sich ein farbloses Öl im Vorlagekolben ab, das in der Kälte kristallisierte und mittels ¹H- und ¹³C-NMR-Spektroskopie als das gewünschte Produkt identifiziert wurde. Ausbeute: 321 mg (0,985 mmol, 71% der Theorie).

### (b) 1,1,3,3-Tetrapropargylaceton

Unter Argon wurden 97,0 mg (0,297 mmol) 2,2,4,4-Tetrapropargylacetondicarbonsäuredimethylester und 343 mg (2,56 mmol) Lithiumiodid in 2,4,6-Collidin 90 min bei 180°C erhitzt. Man ließ abkühlen und gab 5 ml 15%ige Salzsäure zu. Es wurde mit Diethylether überschichtet. Unter kräftigem Rühren gab man solange Wasser zu, bis die wäßrige Phase klar wurde. Die organische Phase wurde abgetrennt, die wäßrige Phase noch dreimal mit Ether extrahiert. Man vereinigte die organischen Phasen und wusch nacheinander mit 5%iger Natronlauge, Wasser und gesättigter Natriumchlorid-Lösung. Es wurde über wasserfreiem Natriumsulfat getrocknet. Am Rotationsverdampfer destillierte man das Lösungsmittel ab, der verbleibende Rückstand erstarrte zu einem farblosen Feststoff. Dieser wurde im Wasserstrahlvakuum destilliert, bei 220°C schieden sich im Vorlagekolben farblose Nadeln ab, die mittels ¹H- und ¹³C-NMR-Spektroskopie als gewünschtes Produkt identifiziert werden konnten. Ausbeute: 38,0 mg (0,180 mmol, 60,6% der Theorie).

### (c) 1,1,3,3-Tetrakis(closo-1,2-dicarbadodecaboranylmethyl)-aceton

Unter Argon wurden 177 mg (0,842 mmol) 1,1,3,3-Tetrapropargylaceton, 542 mg (4,44 mmol) Decaboran (14) und 440 mg (10,2 mmol) trockenes Acetonitril in 5 ml trockenem Toluol 16 h bei 80°C erhitzt, anschließend 4 h bei 100°C und abschließend 4 h bei 120°C erhitzt. Man ließ das Heizbad auf 100°C abkühlen. Überschüssiges Decaboran (14) wurde durch Zugabe einer 1/1 (v/v) Mischung aus konzentrierter Salzsäure und Ethanol und 6-stündigem Erhitzen unter Rückfluß vernichtet. Die organische Phase wurde abgetrennt, die wäßrige wurde noch zweimal mit Toluol extrahiert. Die organischen Phasen wurden vereinigt, mit Wasser gewaschen, über wasserfreiem Natriumsulfat getrocknet und am Rotationsverdampfer eingeengt. Den Rückstand chromatographierte man über Kieselgel mit Petrolether/Essigester im Verhältnis 4/1 und erhielt 87 mg eines farblosen Öls, das in der Kälte kristallisierte. ¹H- und ¹³C-NMR-Spektrum sowie das ESI-Massenspektrum bestätigten die gewünschte Struktur. Ausbeute: 87,1 mg (0,128 mmol, 15,2% der Theorie).

### (d) 2-Allyloxy-1,1,3,3-tetrakis(closo-1,2-dicarbadodecaboranylmethyl)-propan

Unter Argon wurden 5,1 mg Lithiumaluminiumhydrid in trockenem Ether suspendiert und eine Lösung von 21,5 mg (31,5 µmol) 1,1,3,3-Tetrakis(*closo*-1,2-dicarbadodecaboranylmethyl)-aceton in trockenem Ether zugetropft. Man ließ 30 min bei Raumtemperatur rühren und erhitzte anschließend 1 h unter Rückfluß. Man ließ abkühlen und vernichtete überschüssiges Lithiumaluminiumhydrid durch tropfenweises Zugeben von Wasser. Anschließend wurde unter Rühren solange 10%ige Schwefelsäure zugetropft, bis sich alle Niederschläge aufgelöst haben. Man trennte die organische Phase ab und extrahierte die wäßrige Phase noch zweimal mit Ether. Die organischen Phasen wurden vereinigt und mit Wasser gewaschen und über wasserfreiem Natriumsulfat getrocknet. Man dampfte das Lösungsmittel ab und löste den Rückstand in 0,50 ml (6,0 mmol) Allylbromid auf. Dazu gab man eine Lösung von 80 mg (2,0 mmol) Natriumhydroxid und 17 mg (75 µmol) Benzyltriethylammoniumchlorid in 1 ml Wasser und rührte 16 h kräftig bei Raumtemperatur. Anschließend wurde mit 2 ml Wasser verdünnt und 2 ml Ether zugegeben. Man trennte die organische Phase ab und extrahierte die wäßrige Phase zweimal mit Ether. Die organischen Phasen wurden vereinigt, mit Wasser gewaschen und über wasserfreiem Natriumsulfat getrocknet. Man zog das Lösungsmittel am Rotationsverdampfer ab und chromatographierte den Rückstand über Kieselgel mit Petrolether/Essigester im Verhältnis 4/1. Es wurden 6,9 mg eines farblosen hochviskosen Öls isoliert, das durch ¹H- und ¹³C-NMR-Spektroskopie als das gewünschte Produkt identifiziert wurde. Ausbeute: 6,9 mg (9,5 µmol, 30% der Theorie).

## Patentansprüche

1. Bor-haltige Verbindung, die die folgende allgemeine Formel (1) aufweist, 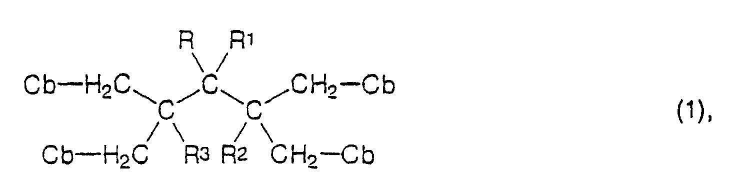 in der
Cb für ein Carboran,
R² und R³ unabhängig voneinander für ein Wasserstoffatom oder einen organischen Rest und
R und R¹ unabhängig voneinander für ein Wasserstoffatom oder eine organische Gruppe stehen oder R und R¹ mit dem Kohlenstoffatom, an das sie gebunden sind, eine Carbonylgruppe bilden.

2. Verbindung nach Anspruch 1, **dadurch gekennzeichnet, daß** das Carboran ein 1,2-Dicarba-*closo*-dodecaboran(12) ist.

3. Verbindung nach Anspruch 1 oder 2, **dadurch gekennzeichnet, daß** an das Carboran ein Spacer und/oder eine löslichkeitsmodulierende Verbindung gebunden ist.

4. Verbindung nach Anspruch 3, **dadurch gekennzeichnet, daß** die löslichkeitsmodulierende Verbindung Glucose ist.

5. Verbindung nach Anspruch 3 oder 4, **dadurch gekennzeichnet, daß** an eines bis an alle Carborane eine löslichkeitsmodulierende Verbindung gebunden ist.

6. Verbindung nach einem der Ansprüche 1 bis 5, **dadurch gekennzeichnet, daß** der organische Rest für R² und R³ eine COOR⁴-Gruppe ist oder zusammen mit den Kohlenstoffatomen, an die sie gebunden sind, einen 6-gliedrigen Ring bilden.

7. Verbindung nach Anspruch 6, **dadurch gekennzeichnet, daß** R² und R³ zur Bildung des 6-gliedrigen Rings ausgewählt sind aus:

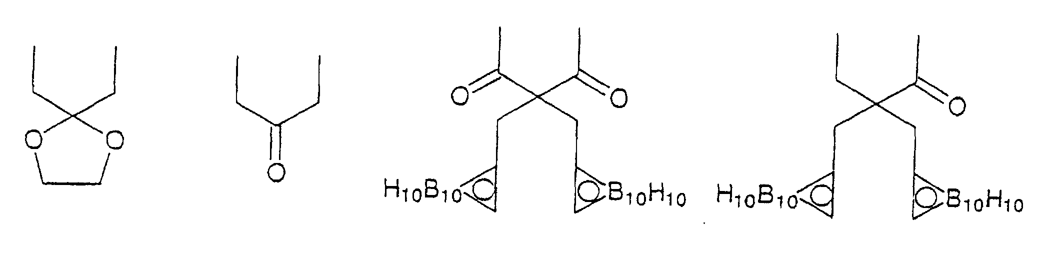

8. Verbindung nach einem der Ansprüche 1 bis 7, **dadurch gekennzeichnet, daß** die für R und/oder R¹ stehende organische Gruppe eine über eine Ether-Brücke gebundene C2 bis C10 Alkyl-Gruppe ist.

9. Verbindung nach Anspruch 8, **dadurch gekennzeichnet, daß** eine Phosphatgruppe an die C2 bis C10 Alkylgruppe gebunden ist.

10. Verbindung nach Anspruch 9, **dadurch gekennzeichnet, daß** ein Mono-, Oligo-, oder Polynukleotid an die Phosphatgruppe gebunden ist.

11. Verfahren zur Herstellung einer Verbindung nach einem der Ansprüche 1 bis 10, **dadurch gekennzeichnet, daß** ein Decaboran (14) mit einer Verbindung der Formel (4) 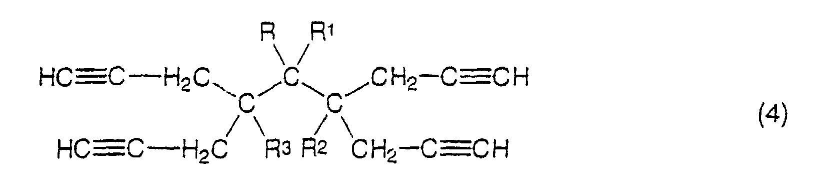 umgesetzt wird.

12. Verwendung einer Verbindung nach einem der Ansprüche 1 bis 10 als Bor-haltigen Marker für die energiefilternden Transmissionselektronenmikroskopie oder für die Bor-Neutroneneinfangtherapie zur Behandlung von Tumorerkrankungen.

## Claims

1. A boron-containing compound which comprises the following general formula (1) 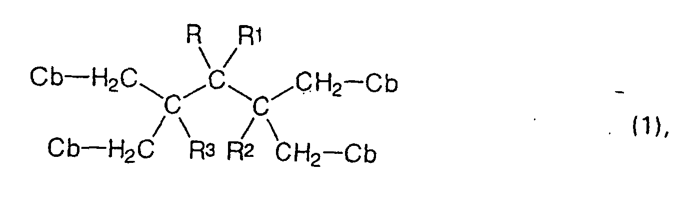 in which
Cb represents carborane,
R² and R³, independent of one another, represent a hydrogen atom or an organic radical, and
R and R¹, independent of one another, represent a hydrogen atom or an organic group, or R and R¹ form a carbonyl group with the carbon atom to which they are bound.

2. The compound according to claim 1, **characterized in that** the carborane is a 1,2-dicarba-*closo*-dodecaborane(12).

3. The compound according to claim 1 or 2, **characterized in that** a spacer and/or a solubility-modulating compound is bound to the carborane.

4. The compound according to claim 3, **characterized in that** the solubility-modulating compound is glucose.

5. The compound according to claim 3 or 4, **characterized in that** a solubility-modulating compound is bound to one up to all carboranes.

6. The compound according to any of claims 1 to 5, **characterized in that** the organic residue for R² and R³ is a COOR⁴ group or together with the carbon atoms to which they are bound form a 6-membered ring.

7. The compound according to claim 6, **characterized in that** for forming the 6-membered ring R² and R³ are selected from the group consisting of:

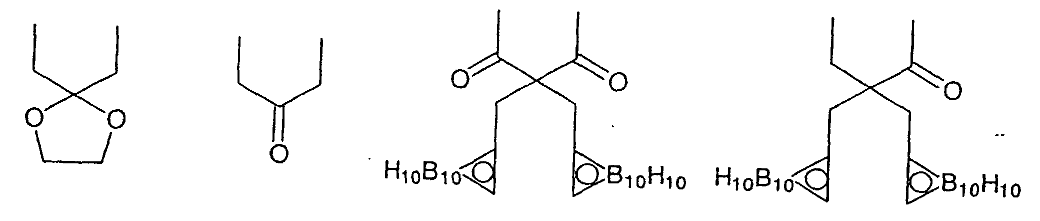

8. The compound according to any of claims 1 to 7, **characterized in that** the organic group denoting R and/or R¹ is a C2 to C10 alkyl group bound via an ether bridge.

9. The compound according to claim 8, **characterized in that** a phosphate group is bound to the C2 to C10 alkyl group.

10. The compound according to claim 9, **characterized in that** a mono-, oligo-, or polynucleotide is bound to the phosphate group.

11. A method of producing a compound according to any of claims 1 to 10, **characterized in that** a decaborane (14) is reacted with a compound of formula (4)

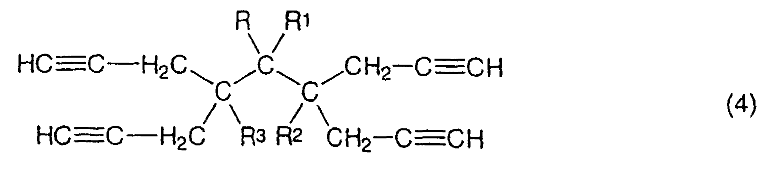

12. Use of a compound according to any of claims 1 to 10 as a boron-containing marker for the energy-filtering transmission electron microscopy or for the boron neutron-capture therapy for treating tumoral diseases.

## Revendications

1. Composé contenant du bore, de formule générale (1) suivante : 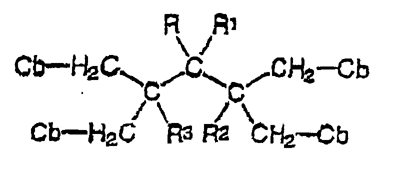 dans laquelle
Cb représente un carborane,
R² et R³ représentent, indépendamment l'un de l'autre, un atome d'hydrogène ou un reste organique et
R et R¹ représentent, indépendamment l'un de l'autre, un atome d'hydrogène ou un groupe organique, ou bien R et R¹ forment un groupe carbonyle conjointement avec l'atome de carbone auquel ils sont liés.

2. Composé suivant la revendication 1, **caractérisé en ce que** le carborane est un 1,2-dicarba-*closo*-dodécaborane(12).

3. Composé suivant la revendication 1 ou 2, **caractérisé en ce qu'**un espaceur et/ou un composé modulateur de solubilité sont liés au carborane.

4. Composé suivant la revendication 3, **caractérisé en ce que** le composé modulateur de solubilité est le glucose.

5. Composé suivant la revendication 3 ou 4, **caractérisé en ce qu'**un composé modulateur de solubilité est lié à l'un, plusieurs ou la totalité des carboranes.

6. Composé suivant l'une des revendications 1 à 5, **caractérisé en ce que** le reste organique représenté par R² et R³ est un groupe COOR⁴ ou bien R² et R³ forment un noyau hexagonal conjointement avec les atomes de carbone auxquels ils sont liés.

7. Composé suivant la revendication 6, **caractérisé en ce que** R² et R³ pour la formation du noyau hexagonal sont choisis entre :

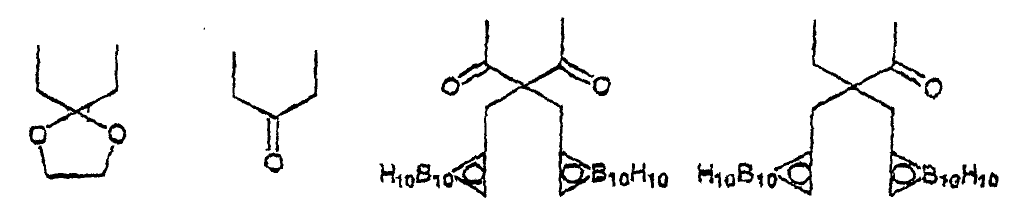

8. Composé suivant l'une des revendications 1 à 7, **caractérisé en ce que** le groupe organique représenté par R et/ou R¹ est un groupe alkyle en C₂ à C₁₀ lié par l'intermédiaire d'un pont éther.

9. Composé suivant la revendications 8, **caractérisé en ce qu'**un groupe phosphate est lié au groupe alkyle en C₂ à C₁₀.

10. Composé suivant la revendication 9, **caractérisé en ce qu'**un mono-, oligo- ou polynucléotide est lié au groupe phosphate.

11. Procédé de production d'un composé suivant l'une des revendications 1 à 10, **caractérisé en ce qu'**on fait réagir un décaborane (14) avec un composé de formule (4)

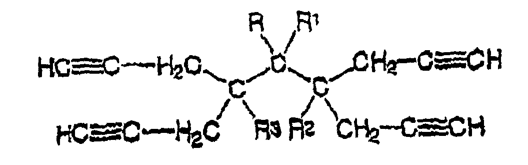

12. Utilisation d'un composé suivant l'une des revendications 1 à 10 comme marqueur contenant du bore en microscopie électronique à transmission avec filtrage d'énergie ou en thérapie au bore par capture de neutrons pour le traitement de maladies tumorales.
